# EUROPÄISCHE PATENTSCHRIFT

(11) **EP 3 310 962 B1**
(45) Veröffentlichungstag und Bekanntmachung des Hinweises auf die Patenterteilung: **02.01.2019**
(21) Anmeldenummer: 16784906.6
(22) Anmeldetag: 21.10.2016
(51) Int. Cl.: D21H 27/00, D21H 27/08

(54) **MARKIERTES FILTERPAPIER ZUM EINSATZ ALS PRÜFSUBSTRAT BEI STANDARDISIERTEN VERFAHREN ZUR BEURTEILUNG DER ZÜNDNEIGUNG VON ZIGARETTEN**
MARKED FILTER PAPER FOR USE AS TEST SUBSTRATE IN STANDARDISED METHODS FOR EVALUATION OF THE IGNITION PROPENSITY OF CIGARETTES
PAPIER FILTRE MARQUÉ DESTINÉ À ÊTRE UTILISÉ COMME SUBSTRAT DE TEST DANS DES PROCÉDÉS NORMALISÉS D'ÉVALUATION DE LA CAPACITÉ DE CIGARETTES À S'ALLUMER

(30) Priorität: 30.10.2015 DE 102015118595
(43) Veröffentlichungstag der Anmeldung: 25.04.2018
(73) Patentinhaber: AXAGARIUS GmbH & Co. KG, 52355 Düren (DE)
(72) Erfinder: MEUSEL, Markus, 52146 Würselen (DE); PROKISCH, Christian, 52355 Düren (DE)
(74) Vertreter: Paul & Albrecht Patentanwälte PartG mbB
(86) Internationale Anmeldenummer: PCT/EP2016/075409
(87) Internationale Veröffentlichungsnummer: WO 2017/072045

(56) Entgegenhaltungen:
- US-A1- 2002 056 535
- US-A1- 2014 322 488

## Beschreibung

Die vorliegende Erfindung betrifft ein Zellulose-Filterpapier zur Überprüfung der Zündneigung von Zigarettenpapier, ein Verfahren zu dessen Herstellung, ein Verfahren zur Überprüfung der Zündneigung von Zigarettenpapier sowie ein Kit mit einer Vielzahl von derartigen Filterpapieren oder Stapeln solcher Filterpapiere.

Unbeaufsichtigte, glimmende Zigaretten gehören laut EU-Kommission in Europa zu den Hauptursachen für Brände mit Todesfolge. Nach Angaben der EU Mitgliedsstaaten für die Jahre 2003 bis 2008 ereigneten sich in der EU jährlich über 30.000 Brände, die durch Zigaretten verursacht wurden. Dabei starben über 1.000 Menschen, mehr als 4.000 wurden verletzt.

Es besteht also ein dringender Bedarf, Gefährdungen durch Zigaretten zu reduzieren. Ein Ansatzpunkt liegt in der Zigarette selbst. So dürfen seit November 2011 in der Europäischen Union nur noch sogenannte Sicherheitszigaretten vertrieben werden. Diese Zigaretten, die bereits seit vielen Jahren in den USA, in Kanada oder Australien verwendet werden, haben eine verminderte Zündneigung durch eine Veränderung des Zigarettenpapieres. So wird das Zigarettenpapier an 2 bis 3 Stellen durch ringförmige Bänder modifiziert, die mit dem bloßen Auge nicht erkannt werden. Wenn die Glut ein solches Band erreicht, steht weniger Sauerstoff zur Verfügung und der Verbrennungsprozess wird reduziert. Die Zigarette kann dann von selbst erlöschen. Beispielhaft für dieses technologische Gebiet ist die US 8,863,757 zu nennen.

Die Fertigung und Prüfung solcher Zigaretten mit verminderter Zündneigung unterliegt engen Sicherheitsnormen. Die DIN EN ISO 12863 (Normprüfverfahren zur Beurteilung der Zündneigung von Zigaretten) legt zum Beispiel Standard-Testmethoden fest.

Dieses Prüfverfahren ermittelt die Wahrscheinlichkeit, ob eine auf einem wärmeabsorbierenden Substrat angeordnete Zigarette ausreichend Wärme erzeugt, um das Abbrennen des Tabakstranges aufrechtzuerhalten und somit möglicherweise einen Brand auszulösen. Als Substrat definiert die Norm eine "horizontale Oberfläche, bestehend aus Filterpapierlagen, auf die eine zu prüfende Zigarette aufgelegt ist". Ganz konkret beschreibt die Norm Zelluloserundfilter mit einem Durchmesser von 15 cm, weiterhin ist das Gewicht des Filters spezifiziert. Standardmäßig wird ein Satz (Stapel) aus 10 Einzelfiltern als Substrat eingesetzt.

Um standardisierte, einheitliche Bedingungen während des Normtestes zu erreichen, ist es notwendig, die raue Oberseite der Filterpapiere nach oben zu orientieren.

Diese Normforderung ist für den Anwender des Verfahrens sehr schwierig umzusetzen. Die Rauigkeit einer solchen Filteroberfläche kann zwar auch messtechnisch (apparativ) untersucht und ermittelt werden, praktisch ist dies jedoch kaum umzusetzen. Deshalb wird die Rauigkeit durch den Anwender in der täglichen Praxis rein haptisch ermittelt. Die Asymmetrie der Filterpapiere mit einer glatteren und einer raueren Seite ist produktionsbedingt, die Unterschiede zwischen den Seiten sind jedoch im Einzelfall auch für den erfahrenen Fachmann sehr schwer zu beurteilen. Welche Seite des Papieres die höhere Rauigkeit aufweist, hängt maßgeblich vom Produktionsverfahren ab. So kann bei der Herstellung von Filterpapier die siebseitige Oberfläche des Filterpapiers die rauere Oberfläche sein, es gibt aber auch Prozesse, bei denen die Siebseite die glattere Seite des späteren Papieres definiert.

Für die Bestimmung der Rauigkeit steht messtechnisch beispielsweise ein Verfahren nach DIN 53108:1995-01 zur Verfügung. Diese Norm beschreibt die Bestimmung der Rauigkeit nach Bendtsen. Die Rauigkeit ist hier der Luftvolumendurchfluss, der zwischen dem Messring des Messkopfes des Bendtsen-Gerätes und der Probenoberfläche hindurchgeht und der bei einem festgelegten Überdruck entsteht. Die Messung der Rauigkeit nach diesem normierten Verfahren ist aufwendig und erfordert eine spezielle Messapparatur.

Das Normverfahren zur Zündfähigkeit fordert, dass die raue Seite des Filterpapieres bei den Bestimmungen nach oben zeigt. Die raue Seite muss also vor dem Einbringen in die Messkammer ermittelt werden, prinzipiell ist dies damit vor oder nach der Vorkonditionierung des Papieres möglich (Vorkonditionierung durch Inkubation des Papiers bei definierter Temperatur und Feuchte), praktischerweise erfolgt dies jedoch vor der Vorkonditionierung, da das vorkonditionierte Filterpapier unmittelbar in die Versuchsapparatur zur Ermittlung der Zündfähigkeit eingesetzt werden muss.

Ein solches Verfahren zur Überprüfung der Zündneigung von Zigarettenpapier umfasst in der Regel die Schritte:
a) Vorkonditionierung eines runden Zellulose-Filterpapiers, das eine raue und eine glatte Oberfläche aufweist;
b) horizontales Auflegen des vorkonditionierten Filterpapieres auf einen Substrathalter (8), wobei die raue Oberfläche des Filterpapieres nach oben zeigt,
c) danach Auflegen eines Metallkranzes (7) auf das Filterpapier, wobei der Metallkranz (7) zwei parallel zueinander beabstandete Metallstifte (9) zur Fixierung einer entzündeten Zigarette aufweist, die in das Kranzinnere hineinragen und zueinander einen Abstand und eine Länge aufweisen, um das nicht entzündete Ende einer Zigarette zu fixieren;
d) anschließend Auflegen einer an einem Ende entzündeten Zigarette mit der Naht des Zigarettenpapiers nach oben auf die raue Oberfläche des Filterpapieres, wobei das nicht entzündete Ende der Zigarette zwischen den Metallstiften (9) fixiert wird und
e) Ermitteln des Abbrennverhaltens der Zigarette.

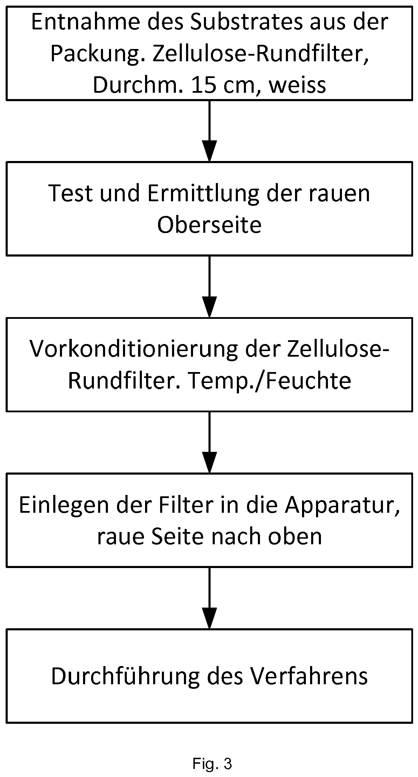

Eine Übersicht über die Verfahrensschritte bei der Bestimmung der Zündneigung von Zigaretten durch den Anwender ist in Fig. 3 dargestellt. Der Stand der Technik hat hier den oben genannten Nachteil, dass die Orientierung des als Substrat eingesetzten Filterpapieres vor der Durchführung des Testes ermittelt werden muss. Die haptische Untersuchung ist unsicher und kann zu Fehlern führen. Dies kann neben Unsicherheiten bei den Versuchsergebnissen letztlich auch weitreichende haftungsrelevante Folgen mit sich bringen. Ein apparatives Überprüfen der Rauigkeit von Oberflächen ist jedoch in der Regel zu zeitaufwendig.

Die Aufgabe der vorliegenden Erfindung bestand darin, ein verbessertes Verfahren zur Überprüfung der Zündneigung von Zigarettenpapier zur Verfügung zu stellen, das eine einfache und zugleich möglichst fehlerfreie Ermittlung der glatten beziehungsweise rauen Seite eines Filterpapieres für die Beurteilung der Zündneigung von Zigaretten ermöglicht und das ohne den Einsatz von Prüfapparaturen auskommt.

Die Aufgabe wird bei einem Verfahren der eingangs genannten Art dadurch gelöst, dass ein markiertes Zellulose-Filterpapier eingesetzt wird, bei dem die Markierung eine einfache und eindeutige Unterscheidung der rauen von der glatten Oberfläche des Zellulosefilterpapiers erlaubt, zweckmäßigerweise auf haptischem Wege und/oder mittels Betrachten durch das Versuchspersonal.

Die vorliegende Erfindung betrifft demnach ein Zellulose-Filterpapier zur Überprüfung der Zündneigung von Zigarettenpapier, das dadurch gekennzeichnet ist, dass das Filterpapier mit einer Markierung versehen ist, mit der die raue von der glatten Oberfläche des Filterpapiers unterschieden werden kann.

Hierzu werden die Zellulosefilter während der Papierproduktion oder nach der Papierproduktion so markiert, dass eine einfache und eindeutige Zuordnung in (rauere) Ober- und (glattere) Unterseite möglich ist.

Demnach ist ein weiterer Gegenstand der vorliegenden Erfindung ein Verfahren zur Herstellung eines erfindungsgemäßen Zellulose-Filterpapiers bei dem das Filterpapier mit einer Markierung versehen wird, mit der die raue von der glatten Oberfläche des Filterpapiers unterschieden werden kann.

Grundsätzlich steht für die Markierung von Papier oder Filtern eine Vielzahl von technischen Möglichkeiten zur Verfügung. Als nicht abschließende Aufzählung seien beispielsweise ein Wasserzeichen, ein halbechtes Wasserzeichen, eine Prägung, ein Druck und/oder eine Stanzung genannt.

Die Markierung ist insbesondere außerhalb des Mittelpunkts des Filterpapiers positioniert, vorzugsweise in einem Randbereich des Filterpapiers. Dies ist von Vorteil, weil damit sichergestellt wird, dass die Markierung das Abbrennverhalten einer während des Tests aufgelegten Zigarette nicht beeinflusst beziehungsweise im Auflagebereich der Zigarette homogene Eigenschaften besitzt.

Vorzugsweise wird die Markierung so ausgewählt, dass diese eine Unterscheidung der rauen von der glatten Oberfläche des Filterpapieres auf haptischem Wege und/oder mit dem (normalsichtigen) menschlichen Auge erlaubt. Das heißt, dass die Markierung vom Versuchspersonal ohne besonderes Training und ohne weitere Hilfsmittel imstande ist, die raue Seite des Filterpapiers von der glatten zu unterscheiden.

Dabei kann die Markierung des Filterpapiers beispielsweise die raue Oberfläche des Filterpapiers kennzeichnen, dann weiß das Personal, dass diese Seite für das Testverfahren oberseitig positioniert sein muss. Da die Stapel für den Test auch mit der rauen Seite nach oben zusammengestellt werden müssen, hat dies gewisse Vorteile für den Arbeitsablauf. Selbstverständlich kann aber auch die glatte Seite des Filterpapiers markiert sein.

Als Markierung kann beispielsweise ein vorzugsweise unsymmetrisches Symbol verwendet werden, wobei die Markierung insbesondere mindestens zwei geometrische Symbole umfasst oder daraus besteht, die sich vorzugsweise in ihrer Form, Farbe und/ oder Größe voneinander unterscheiden. Ein unsymmetrisches Symbol ist dann bevorzugt, wenn die Markierung auf beiden Seiten des Filterpapieres erkennbar ist. Eine derartige Kennzeichnung kann beispielsweise ein Pfeil und ein Punkt (z.B. ► ●) umfassen, die vertikal oder schräg zu einer durch den Kreismittelpunkt des Rundfilters angeordnet sind. Das Zeichen ist dann sowohl von der Ober-, als auch von der Unterseite her zu betrachten, allerdings kann man die Orientierung des Pfeils (z. B. links vom Punkt) der oberen Papierseite zuordnen. Wären sie hingegen auf der durch den Kreismittelpunkt des Rundfilters verlaufenden Linie angeordnet, würde sich kein Unterschied zwischen Ober- und Unterseite des Filterpapiers ergeben.

Eine Möglichkeit, eine Markierung während der Papierproduktion einzubringen, ist ein Wasserzeichen, das z. B. in der Siebpartie einer Papiermaschine eingebracht ist. Bei den echten Wasserzeichen wird die Papierbahn durch eine fest positionierte rotierende Walze auf der Siebpartie verdünnt oder verdichtet. Dabei können Schriftzüge wie "rau", "oben", "rough", "upside" oder "top" und/oder einzelne, gegebenenfalls unsymmetrische Symbole verwendet werden. Bei diesem Verfahren der Markierung ist eine vorherige Bestimmung der rauen und weniger rauen Seite der entstehenden Papierbahnen nicht nötig, da aufgrund des Herstellungsprozesses der Papierbahn die beiden unterschiedlichen Oberflächen bereits definiert sind.

Alternativ zu der Markierung per Wasserzeichen können auch halbechte Wasserzeichen durch Einpressen in die bereits wesentlich trockenere Papierbahn nach dem Verlassen der Siebpartie entstehen. Durch geeignete Formgebung des Wasserzeichens kann auch hier später im Papier leicht zwischen den asymmetrischen Seiten unterschieden werden. Auch bei diesem Verfahren der Markierung ist eine vorherige Bestimmung der rauen und weniger rauen Seite der entstehenden Papierbahnen nicht zwangsweise nötig, da aufgrund des Herstellungsprozesses der Papierbahn die beiden unterschiedlichen Oberflächen bereits definiert sind.

Insbesondere im Hinblick auf ein späteres Zuschneiden von Papierbahnen bei diesen Verfahren und anderen Verfahren, bei denen der Zuschnitt nach dem Anbringen der Markierungen erfolgt, sollte darauf geachtet werden, dass die Markierungen in der Weise auf die Oberfläche der Papierbahn angebracht sind, dass sie nach dem Zuschneiden der Papierbahn in kleinere Segmente, z. B. Kreise (Rundfilter), auf jedem der entstehenden Segmente sichtbar ist.

Die Markierung kann auch durch Anbringen einer Prägung erfolgen. Beim Prägen wird durch ein Umformwerkzeug Druck auf die Oberfläche eines Zellulosefilterpapieres ausgeübt, der auf der Papieroberfläche eine Verformung zu einem Relief bewirkt. Auch hier können asymmetrische Formen, Muster oder Schriftzüge eingeprägt werden, welche die Identifizierung der Oberseite erlauben. Vorzugsweise erfolgt die Prägung in die trockene Papierbahn nach dem Verlassen der Siebpartie, so dass produktionsbedingt die rauere Oberfläche bekannt ist. Hier ist eine Bestimmung der Rauigkeit der beiden Oberflächen der Papierbahn nicht notwendigerweise erforderlich, kann jedoch aus Sicherheitsgründen jederzeit durchgeführt werden. Der Vorteil hierbei liegt darin, dass bei einer Papierrolle die raue Seite nur einmal bestimmt werden muss, um die Orientierung vieler tausend Filterpapiere sicherzustellen.

Eine weitere Möglichkeit zur Erzeugung einer Markierung erfolgt durch Stanzen in die trockene Papierbahn nach dem Verlassen der Siebpartie. Durch geeignetes Stanzen kann entweder ein Schriftzug wie "oben" die rauere Seite markieren (auf der glatteren, "unteren" Seite wäre der entsprechende Schriftzug nur in Spiegelschrift sichtbar), oder die raue Seite kann durch ein vorzugsweise unsymmetrisches Symbol oder eine Anordnung mit mindestens zwei unterschiedliche Symbole mit unterschiedlicher Größe und/oder unterschiedlicher Form.

Auch das Aufbringen einer Markierung mittels Druck ist möglich, z. B. eines Schriftzuges oder mindestens eines Zeichens (symmetrisch oder unsymmetrisch, einfarbig oder mehrfarbig) oder mindestens zweier unterschiedlicher Symbole. Die Symbole können sich beispielsweise in ihrer Größe, Form und/oder Farbe unterscheiden.

Die Markierung kann in einer Ausführungsform der vorliegenden Erfindung beim Herstellungsprozess des Papiers, auf der Ebene der Rollenware, der geschnittenen Bögen oder auf der Ebene der fertig gestanzten oder geschnittenen Filterpapiere stattfinden.

Insofern wird nach einer ersten Ausgestaltung des erfindungsgemäßen Verfahrens zur Herstellung des erfindungsgemäßen Filterpapiers das Filterpapier noch während der Herstellung auf der Siebseite der Siebpartie markiert, beispielsweise mittels eines echten Wasserzeichens. Die Wasserzeichen sind dabei zweckmäßigerweise in der Weise angeordnet, dass sie nach dem Durchlaufen des gesamten Produktionsprozesses bis hin zum fertigen Rundfilter-Papier an der gewünschten Stelle jedes einzelnen Filterpapieres angeordnet sind.

Nach einer zweiten Ausführungsform des erfindungsgemäßen Verfahrens zur Herstellung des erfindungsgemäßen Filterpapiers wird das Filterpapier dem Markierungsprozess als Rollenware zugeführt. Bei der Rollenware ist die Lage der rauen Oberfläche entweder vom Produktionsprozess her schon bekannt oder kann mithilfe eines Messverfahrens, beispielsweise gemäß DIN 53108:1995-01 (Januar 1995) einmalig für die gesamte Rolle bestimmt werden, sodass die Orientierung für den weiteren Produktionsprozess bekannt ist. Die Markierung erfolgt dann an der Rollenware beispielsweise durch Stanzen, Prägen oder Drucken an denjenigen Positionen, an denen später bei dem fertigen Rundfilter die Markierung gewünscht ist. Anschließend kann die markierte Rollenware zu Bögen geschnitten werden, von denen dann die Rundfilter ausgestanzt werden können.

Üblicherweise werden aus dem produzierten Papier als Rollenware im ersten Schritt Bögen geschnitten, die in einem weiteren Schritt auf die erforderliche Formen und Maße (Endmaß) (z.B. 15 cm Rundfilter) gestanzt/geschnitten werden.

Bevorzugt erfolgt das Schneiden der Rollenware in Bögen in einer Form, dass die Orientierung der raueren Oberfläche der Bögen aufgrund der Aufrollung des Filterpapieres bei der Herstellung noch bekannt ist, so dass keine zusätzliche Messung der Oberflächenrauigkeiten vorgenommen werden muss, um die korrekte Markierung der rauen Oberfläche des Filterpapiers zu gewährleisten.

Man kann jedoch der Sicherheit halber vor der Markierung der rauen Oberfläche eines Filterpapierbogens die Rauigkeit der beiden Oberflächen des Filterpapiers nochmals bestimmen. Die Positionierung der Markierungen wird dann in der Weise vorgenommen, dass nach dem Stanzen eines Bogens in das Endmaß eines Filterpapiers die Markierung auf jedem Endmaß an gewünschter Stelle befindlich ist.

Damit erfolgt nach einer dritten Ausgestaltung des erfindungsgemäßen Verfahrens zur Herstellung des erfindungsgemäßen Filterpapiers die Markierung nach dem Zuschneiden der Rollenware zu Bögen. Auch in diesem Fall ist die Lage der rauen Oberfläche entweder vom Produktionsprozess her bekannt oder kann für die gesamte Rolle vor dem Zuschnitt zu Bögen bestimmt werden. Wenn Bögen angeliefert werden, kann die raue Oberfläche auch für jeden Bogen einzeln bestimmt werden, beispielsweise gemäß DIN 53108:1995-01 (Januar 1995). Auf den Bögen können die jeweiligen Markierungen durch Stanzen, Prägen oder Drucken an entsprechend gewünschter Position der später erzeugten Rundfilter angebracht werden. Die Rundfilter können anschließend aus den Bögen ausgestanzt und verpackt werden.

Das Anbringen der Markierung kann auch erst bei bzw. nach dem Stanzen oder Schneiden der Bögen auf die gewünschten Endmaße der Segmente erfolgen. Auch hier erfolgt bevorzugt das Schneiden der Rollenware in Bögen und das anschließende Stanzen der Bögen auf das gewünschte Endmaß eines Filterpapiers in einer Form, dass die Orientierung der raueren Oberfläche der Endmaße aufgrund der Aufrollung des Filterpapieres bei der Herstellung noch bekannt ist, so dass keine weitere Messung der Oberflächenrauigkeiten vorgenommen werden muss, um die Markierung der raueren Oberfläche des Filterpapiers zu gewährleisten.

Damit wird nach einer vierten Ausführungsform des erfindungsgemäßen Verfahrens zur Herstellung des erfindungsgemäßen Filterpapiers die Markierung der Filterpapiere vorgenommen, nachdem diese aus den Bögen ausgestanzt sind. Auch in diesem Falle ist die Lage der rauen Seite der in den Produktionsprozess eingeschleusten Rollenware entweder von der Herstellung bekannt oder kann zuvor anhand eines einzigen Versuchs für die gesamte Papierrolle bestimmt werden, beispielsweise gemäß DIN 53108:1995-01 (Januar 1995). Die Markierung der gestanzten Rundfilter kann über Stanzen, Prägen oder Drucken erfolgen. Da die Lage der rauen Oberfläche über den gesamten Produktionsprozess bekannt ist, ist es hierbei nicht erforderlich, vor dem Anbringen der Markierung die raue Oberfläche eines jeden Rundfilters zu bestimmen. Das Anbringen der Markierung auf gestanzten Rundfiltern hat den Vorteil, dass die Lager der Markierung(en) mit einer höheren Präzision definiert werden kann als bei den anderen vorgenannten Verfahren.

Wenn nötig erfolgt die Bestimmung der Rauigkeit der beiden Oberflächen eines Filterpapiers wie vorstehend ausgeführt wurde bevorzugt durch ein Verfahren nach DIN 53108:1995-01 (Januar 1995). Diese Norm beschreibt die Bestimmung der Rauigkeit nach Bendtsen. Die Rauigkeit ist hier der Luftvolumendurchfluss, der zwischen dem Messring des Messkopfes des Bendtsen-Gerätes und der Probenoberfläche hindurchgeht und der bei einem festgelegten Überdruck entsteht.

Bedingt durch den Einsatz in standardisierten Verfahren zur Ermittlung der Zündfähigkeit von Zigaretten, bietet es sich an, die Markierung bevorzugt auf einem Rundfilter aus Zellulosefilterpapier innerhalb eines 1,5 cm breiten Streifens vom Rand des Rundfilters aus anzubringen, mehr bevorzugt eines 1,2 cm breiten, weiter bevorzugt eines 1 cm breiten, um das Testsubstrat (das Filterpapier, bevorzugt ein Rundfilter) für die aufliegende Zigarette nicht zu beeinflussen.

Bevorzugt handelt es sich bei dem Filterpapier um einen einlagigen Rundfilter mit einem Durchmesser im Bereich von 130 mm bis 170 mm, insbesondere von (150 ± 10) mm, weiter bevorzugt ein Rundfilter mit einem Durchmesser von (150 ± 5) mm, besonders bevorzugt mit einem Durchmesser im Bereich von (150 ± 2) mm.

Die Markierung der raueren Oberfläche kann zum einen durch eine Markierung auf der raueren Oberfläche erfolgen, wobei diese Markierung nur von der raueren Seite des Filterpapiers und nicht von der glatteren Seite aus sichtbar ist (z. B. durch Wasserzeichen, Prägung oder Druck). Damit ist die raue Seite unmittelbar identifizierbar. Bevorzugt wird derartig markiertes Filterpapier entweder mit einer Zeichenfolge versehen, die eine sofortige Zuordnung ermöglicht (wie z. B. "rau", "raue Seite", "oben" oder dergleichen) oder ein derartig markiertes Filterpapier wird mit einer Information geliefert, dass die (z. B. durch ein Wasserzeichen oder einen Druck) markierte Oberfläche eines Filterpapieres die rauere Oberfläche des Filterpapiers ist. Diese Information kann in Form eines digitalen Mediums, einer schriftlichen Notiz etc. geliefert werden.

Alternativ kann eine Markierung auf beiden Seiten des Filterpapiers sichtbar sein, z. B. durch Wasserzeichen, Prägung, Druck oder Stanzung. Dem Fachmann kann so ebenfalls direkt die Information geliefert werden, welche der beiden Seiten eines Filterpapiers die Seite mit der raueren Oberfläche und welches die Seite mit der glatteren Oberfläche ist. Dies kann z. B. in Form von Schriftzeichen wie "raue Seite" oder "oben" Markierung für die rauere Seite erfolgen, die nur richtig gelesen werden kann, wenn der Fachmann auf die entsprechend rauere Seite eines Filterpapiers schaut. Wird hingegen von der glatteren" Seite aus auf diese Zeichenfolge geguckt, so erscheint sie spiegelverkehrt. Somit ist bei Verwendung von ausgestanzten Schriftzügen wie z. B. "oben" oder "rau" keine weiter Information nötig, da der Fachmann aufgrund der Lesbarkeit/Unlesbarkeit der Zeichenfolge direkt die richtige Orientierung/Rauigkeit des Filterpapiers ermitteln kann. Wird ein Druck verwendet, so kann die glatte Seite beispielsweise mit "unten" oder "glatt" beschriftet sein.

Ein einzelnes, insbesondere unsymmetrisches Symbol bzw. dessen Orientierung, das sich nicht im Mittelpunkt einer Filterpapieroberfläche befindet, kann ausreichen um eine einfache Unterscheidung zwischen rauerer und glatterer Oberfläche eines Filterpapiers zu ermöglichen. Bevorzugt befindet sich ein solches Symbol vollständig innerhalb eines 1,5 cm breiten Streifens vom Rand des Rundfilters aus anzubringen, mehr bevorzugt eines 1,2 cm breiten, weiter bevorzugt eines 1 cm breiten, um das Testsubstrat (das Filterpapier, bevorzugt ein Rundfilter) für die aufliegende Zigarette nicht zu beeinflussen.

Unter einem unsymmetrischen Symbol wird beispielsweise ein Symbol verstanden, das an einer durch das Symbol und den Mittelpunkt des Rundfilters vertikal verlaufende Spiegelebene keine Spiegelsymmetrie besitzt.

Im Fall eines Rundfilters mit einem unsymmetrischen Symbol als Markierung muss diese zu erkennen geben, welche Orientierung des unsymmetrischen Symbols zum nächstgelegenen Randbereich des Filterpapiers die rauere (und damit auch im Umkehrschluss die glattere) Oberfläche eines Filterpapiers definiert. Bevorzugt wird diese Information in Form einer Abbildung der raueren Oberfläche mit der Orientierung des unsymmetrischen Symbols auf dem Rundfilter und dem Hinweis, dass es sich bei dieser Ansicht um die Ansicht auf die rauere (oder glattere) Oberflächenseite eines Rundfilters handelt, beispielsweise als Informationsblatt oder elektronisch bereit gestellt. Bei einem unsymmetrischen Symbol kann es sich z. B. um einen Pfeil der ein Dreieck handeln.

Bei einem rechteckigen Filter gelten die gleichen Voraussetzungen wie für einen Rundfilter, jedoch muss hier die Unsymmetrie des Symbols naturgegeben nicht an dem Radius, also der durch den Kreismittelpunkt verlaufenden Linie, sondern an dem Lot zur Kante des Filterpapiers, dass durch die Mitte des ausgestanzten Symbols geht, gegeben sein.

Im Fall eines Rundfilters mit zwei oder mehr geometrischen Symbolen als Markierung können sich vorzugsweise zumindest zwei oder alle von diesen voneinander unterscheiden. Damit bei dieser Ausgestaltung eine Unterscheidung der rauen von der glatten Oberfläche des Filterpapiers möglich ist, kann die Anordnung der beiden Symbole zueinander derart ausgestaltet sein, dass die Mittelpunkte beider Symbole nicht auf einer gedachten Linie vom Mittelpunkt zum Randbereich des Filterpapiers liegen um eine Unterscheidung der beiden Seiten zu ermöglichen, wenn beide Symbole von beiden Seiten aus sichtbar sind. Bevorzugt liegen die beiden Symbole jeweils maximal 2 cm voneinander entfernt und jeweils maximal 1,5 cm, bevorzugt maximal 1 cm, vom Randbereich eines Filterpapiers entfernt.

Besonders bevorzugt wird diese Information in Form einer Abbildung der raueren Oberfläche eines Rundfilters mit der Orientierung der beiden Symbole auf dem Rundfilter und dem Hinweis, dass es sich bei dieser Ansicht um die Ansicht auf die rauere Oberflächenseite eines Rundfilters handelt, übermittelt. So kann z. B. eine auf einem Informationsblatt oder einer Datei beigefügten Abbildung die entsprechende Erläuterung gegeben werden. Bei einem rechteckigen Filter gelten hier die gleichen Voraussetzungen zur Bestimmung der rauen Oberflächenseite wie bei einen Rundfilter.

Ein ausgestanztes Symbol kann direkt am Randbereich des Filterpapiers ausgestanzt sein oder mindestens 0,5 mm, bevorzugt mindestens 1 mm, mehr bevorzugt mindestens 2 mm vom Randbereich des Filterpapiers entfernt. Bevorzugt befindet sich die Ausstanzung eines Symbols zur Markierung einer rauen oder einer glatteren Oberfläche eines Filterpapiers mindestens 0,5 mm vom Randbereich und maximal 1,5 cm vom Randbereich eines Rundfilters entfernt. Werden mehrere ausgestanzte Symbole verwendet, gelten die vorgenannten Werte entsprechend.

Vorzugsweise betragen maximale Länge und maximale Breite eines ausgestanzten Symbols unabhängig voneinander maximal 1,4 cm, weiter bevorzugt maximal 0,8 cm.

Ein weiterer Gegenstand der vorliegenden Erfindung betrifft ein Verfahren zur Überprüfung der Zündneigung von Zigarettenpapier umfassend die Schritte:
a) Vorkonditionierung eines runden Zellulose-Filterpapiers, das eine raue und eine glatte Oberfläche aufweist;
b) horizontales Auflegen des vorkonditionierten Filterpapieres auf einen Substrathalter (8), wobei die raue Oberfläche des Filterpapieres nach oben zeigt,
c) danach Auflegen eines Metallkranzes auf das Filterpapier, wobei der Metallkranz zwei parallel zueinander beabstandete Metallstifte zur Fixierung einer entzündeten Zigarette aufweist, die in das Kranzinnere hineinragen und zueinander einen Abstand und eine Länge aufweisen, um das nicht entzündete Ende einer Zigarette zu fixieren;
d) anschließend Auflegen einer an einem Ende entzündeten Zigarette mit der Naht des Zigarettenpapiers nach oben auf die raue Oberfläche des Filterpapieres, wobei das nicht entzündete Ende der Zigarette zwischen den Metallstiften fixiert wird und
e) Ermitteln des Abbrennverhaltens der Zigarette,
wobei das Verfahren dadurch gekennzeichnet ist, dass das Filterpapier ein erfindungsgemäßes Zellulose-Filterpapier ist, also ein solches, das mit einer Markierung versehen ist, mit der die raue von der glatten Oberfläche des Filterpapiers unterschieden werden kann.

Für das erfindungsgemäße Überprüfungs-Verfahren wird in der Regel eine rechteckige Prüfkammer aus einem starren, durchsichtigen Werkstoff mit einem Rauchabzug verwendet, der mittig auf der Kammeroberseite angebracht ist. Bevorzugt sind die Maße dieser Kammer (340 ± 25) mm Höhe, (292 ± 6) mm Breite und (394 ± 6) mm Tiefe. Bevorzugt sind die Maße des Rauchabzuges (165 ± 13) mm Höhe und (152 ± 6) mm Innendurchmesser.

In der Prüfkammer ist ein herausnehmbarer zylindrisch ausgestalteter Substrathalter für das Filterpapier und die zu prüfende Zigarette vorgesehen. Vorzugsweise besitzt der Substrathalter eine auf die Größe von Zigaretten abgestimmte Dimensionierung. Typischerweise weist der Substrathalter einen Außendurchmesser von 165 ± 1 mm, einen Innendurchmesser von 127 ± 1 mm und damit eine Wandstärke von etwa 38 mm auf. Die Höhe liegt bei 50 ± 1 mm. An seiner Oberkante weist der Substrathalter eine zur Innenseite abfallende umlaufende Stufe mit einer Tiefe von 10 ± 2,5 mm auf. Die Stufe ist konzentrisch angebracht und besitzt einen Durchmesser von 152 ± 1 mm. Dadurch entsteht ein treppenartiger Absatz, dessen tiefer liegende Fläche dem Filterpapier als umlaufende randseitige Auflage dient. Die umlaufende Auflage besitzt eine Tiefe von etwa 25 mm.

Um das Filterpapier oder den Stapel von Filterpapieren zu fixieren ist ein Metallkranz vorgesehen, der von oben auf den Rand des in den Halter gelegten Filterpapiers aufgesetzt wird. Der Metallkranz besitzt einen Außendurchmesser, der kleiner als der Innendurchmesser der Stufe entspricht, also vorliegend etwa 150 ± 1 mm. Der Innendurchmesser des Kranzes ist zweckmäßigerweise so gewählt dass der Metallkranz nach dem Auflegen höchstens bündig mit der Innenwandung des Substrathalters abschließt, vorzugsweise ist der Innendurchmesser jedoch größer, sodass der Metallkranz umlaufend von der Innenwand des Substrathalters zurückspringt, also beispielsweise 130 ± 1 mm. Die Materialdicke des Metallkranzes liegt bei etwa 6,4 ± 1 mm.

Der Metallkranz besteht vorzugsweise aus einem Metall mit einem hohen spezifischen Gewicht, also beispielsweise einer Dichte von 8,550 ± 150 kg*m-3, bevorzugt aus Messing, damit die Filterpapierblätter durch das Gewicht des Metallkranzes flach aufeinander gepresst werden. Die Masse des Metallkranzes liegt beispielsweise bei 235 g bis 295 g.

In seitlicher Schnittansicht sind in dem Metallkranz zwei zueinander parallel beabstandete Metallstifte eingelassen, die sich etwa 17 ± 1 mm in das Innere des Metallkranzes erstrecken. Die Metallstifte sind dabei so positioniert, dass eine mittig zwischen und parallel zu den Metallstiften verlaufende gedachte Linie durch den Mittelpunkt des Metallkranzes verläuft. Der Abstand der Metallstifte zueinander ist so gewählt, dass eine Zigarette, die zwischen den Stiften eingelegt wird, am seitlichen Wegrollen gehindert, also fixiert ist. Bei einer üblichen Zigarette mit einem Durchmesser von 8 mm beträgt der Abstand der Metallstifte etwa 8,1 ± 1 mm.

Bevorzugt befindet sich der Boden des Substrathalter 20 ± 1 mm über dem Boden der vorgenannten Prüfkammer. Hierdurch kann eine ausreichende Luftzufuhr auch unterhalb des eingelegten Filterpapiers sichergestellt werden. Dieser Abstand kann beispielsweise durch drei oder vier Füße realisiert werden, die als Abstandhalter dienen und an der Unterseite des Substrathalters in vorzugsweise identischen radialen Abständen fixiert sind. Die Füße können stattdessen auch an entsprechender Position mit der Prüfkammer fest verbunden sein.

Nach einer bevorzugten Weiterbildung des vorgenannten Überprüfungs-Verfahrens werden die Ergebnisse des Schrittes e) nach folgenden Kriterien ausgewertet:
A) vollständiges Abbrennen der Zigarette;
B) unvollständiges Abbrennen der Zigarette.

Dabei bedeutet A), dass eine Zigarette weiterbrennt, bis die Vorderkante des Mundstückpapiers bei Filterzigaretten oder bei Zigaretten ohne Filter die Metallstifte erreicht sind und B), dass die Zigarette erlischt, bevor die Vorderkante des Mundstückpapiers bei Filterzigaretten oder bei Zigaretten ohne Filter die Metallstifte erreicht sind.

Zur Verbesserung der Aussagekraft der Versuche kann vorgesehen sein, das Überprüfungsverfahren mit einer Vielzahl von Zigaretten gleichen Typs, das heißt vor allem mit gleichem Zigarettenpapier, zu wiederholen. Demnach kann das Verfahren vorzugsweise die folgenden weiteren Schritte umfassen:
f) wenigstens 39 maliges Wiederholen der Schritte a) bis e) und
g) Ermitteln des Anteils der wenigstens 40 Bestimmungen, bei denen das Ergebnis A) lautet.

Bei dem Überprüfungsverfahren kann grundsätzlich ein einzelnes Filterpapier zur Auflage der entzündeten Zigarette dienen, wobei vorzugsweise jedoch ein Stapel aus mit einer Definierten Stückzahl gleicher Filterpapiere eingesetzt wird. Bei einem solchen Verfahren kann somit im Schritt a) eine Vielzahl von Filterpapieren vorkonditioniert und im Schritt b) ein Stapel der vorkonditionierten Filterpapiere auf den Substrathalter aufgelegt werden, wobei die raue Oberfläche der Filterpapiere des Stapels nach oben zeigen und der Stapel wenigstens 3, insbesondere 10 oder 15 Filterpapiere umfasst. Weiter bevorzugt werden die wenigstens 40 Bestimmungen jeweils einmal mit einem Stapel von 10 und danach jeweils noch mit Stapeln von 3 und von 15 Filterpapieren durchgeführt, um das Abbrennverhalten in Relation zur Zahl der Filterpapierlagen zu untersuchen.

Die zu untersuchende Zigarette wird typischerweise vor Anzünden bei 5 ± 1 mm und 15 ± 1 mm vom anzuzündenden Zigarettenende ausgehend markiert, beispielsweise mit einem Bleistift oder einem anderen Markierungsgerät. Anschließend wird die Zigarette entzündet und erst dann auf die raue Oberfläche des eingespannten Filterpapiers beziehungsweise den Filterpapierstapel gelegt, wenn die Glut die 15 ± 1 mm Markierung ausgehend vom angezündeten Zigarettenende erreicht.

Die Auswertung des Schrittes e) wird bevorzugt mit einem höheren Detaillierungsgrad vorgenommen. Dabei werden zusätzlich zu den Ergebnissen A) vollständiges Abbrennen und B) unvollständiges Abbrennen die beiden weiteren Ergebnisse C) und D) untersucht, mit
C) Selbstlöschung, wenn die Zigarette nach dem Anzünden erlischt, bevor die 15 ± 1 mm vom anzuzündenden Zigarettenende ausgehende Markierung der Zigarette erreicht ist, wird als A) gewertet mit dem Vermerk, dass dies vor dem Auflegen auf die raue Oberfläche des Zellulosefilterpapiers geschehen ist;
D) Wenn der Rauch der abbrennenden Zigarette auch nach Überprüfung der Kammer und des Abluftsystems nicht laminar ist, wird dieses Ergebnis festgehalten.

Vorzugsweise wird bei dem erfindungsgemäßen Verfahren jede dem Test zugeführte Zigarette vor dem Anzünden vorkonditioniert, indem diese mindestens 24 h vor der Prüfung in einer Umgebung mit einer relativen Luftfeuchtigkeit von 55 ± 5 % und einer Temperatur von 23 ± 3 °C aufbewahrt wird. Hierdurch können Schwankungen im Abbrennverhalten kompensiert werden, die aus einer möglichen unterschiedlichen Vorlagerung herrühren könnten.

Die Vorkonditionierung des oder der Filterpapiere wird beispielsweise so vorgenommen, dass eine 100-Blatt-Schachtel mit markierten Filterpapier bei offenem Deckel für mindestens sieben Tage (7 * 24 h) vor der Prüfung in einer Umgebung mit einer relativen Luftfeuchtigkeit von 55 ± 5 % und einer Temperatur von 23 ± 3 °C aufbewahrt wird. Alternativ können bei der Verwendung von Filterpapierstapeln dementsprechende Sets, also Stapel von 3, 10 oder 15 markierten Filterpapieren für mindestens 8 h vor der Prüfung in einer Umgebung mit einer relativen Luftfeuchtigkeit von 55 ± 5 % und einer Temperatur von 23 ± 3 °C aufbewahrt werden. Besonders bevorzugt werden Sets mit 15 markierten Filterpapieren verwendet, die für mindestens 8 h vor der Prüfung in einer Umgebung mit einer relativen Luftfeuchtigkeit von 55 ± 5 % und einer Temperatur von 23 ± 3 °C aufbewahrt werden.

Eine weitere bevorzugte Ausführungsform richtet sich auf ein erfindungsgemäßes Verfahren, bei dem die mittlere Masse und die Standardabweichung von 5 Sätzen mit je 15 Blättern des konditionierten, markierten Filterpapiers 26,1 ± 0,5 g betragen, wobei die Standardabweichung der fünf Wägungen nicht mehr als 0,3 g beträgt.

Eine weitere bevorzugte Ausführungsform richtet sich auf ein erfindungsgemäßes Verfahren, bei dem die mittlere Masse von 5 Sätzen mit je 15 getrockneten markierten Filterpapieren 24,7 ± 0,5 g beträgt, wobei die Standardabweichung der fünf Wägungen nicht mehr als 0,3 g beträgt und die Trocknung jeweils eines Satzes mit 15 Blättern für mindestens 16 h bei 60 ± 2 °C erfolgt ist und die Abkühlung auf 23 ± 3 °C in einem dicht geschlossenen Behälter vorgenommen wurde, dessen Volumen maximal 30mal größer ist als das Volumen des Satzes mit 15 Blättern.

Eine weitere bevorzugte Ausführungsform richtet sich auf ein erfindungsgemäßes Überprüfungs-Verfahren, bei dem der Versuchsaufbau dem der DIN EN ISO 12863 aus 2010 oder der DIN EN ISO 12863:2011-04 vom April 2011 entspricht. Ebenso kann das Verfahren nach anderen Prüfverfahren durchgeführt werden, die den Einsatz von Zellulosefilterpapieren vorsehen, insbesondere andere Prüfverfahren, die auf der DIN EN ISO 12863 basieren.

Die vorliegende Erfindung betrifft weiterhin ein Kit umfassend eine Vielzahl von erfindungsgemäßen Filterpapieren oder wenigstens einen Stapel von 3, 10 oder 15 solcher Filterpapiere, eine Information, wie die raue Oberfläche des Zellulose-Filterpapieres anhand der Markierung zu ermitteln ist und optional eine Versuchsdurchführung der DIN EN ISO 12863 von 2010 und/ oder DIN EN ISO 12863:2011-04.

Bei dem Kit ist vorzugsweise die Ausrichtung aller rauen Oberflächen der Zellulose-Filterpapiere eines Stapels identisch. Damit kann der Stapel vom Versuchspersonal in einem Arbeitsschritt in die Prüfapparatur eingesetzt werden.

Bei dem Kit kann ferner vorgesehen sein, dass darin wenigstens 5 oder wenigstens 10 Stapel enthalten sind.

Bei dem Kit kann beispielsweise dann auf das Beifügen einer Information, wie die raue Oberfläche des Zellulose-Filterpapieres anhand der Markierung zu ermitteln ist, dann verzichtet werden, wenn sich dies bereits aus der Ausgestaltung der Markierung selbst ergibt. Also beispielsweise dann, wenn die Filterpapiere auf der rauen Oberfläche beispielsweise den Aufdruck "rau" oder "oben" oder gleichbedeutende Begriffe in deutscher, englischer oder einer anderen Sprache aufweisen. Dennoch kann auch in solchen Fällen sicherheitshalber eine entsprechende Information dem Kit beiliegen, beispielsweise in gedruckter Form oder auf einem Datenträger.

### Beispiele:

Die vorliegende Erfindung wird im Folgenden anhand von acht Zeichnungen und Ausführungsbeispielen näher erörtert. Darin zeigt
- Fig. 1: den Aufbau einer Prüfkammer zur Beurteilung der Zündneigung von Zigaretten in Darstellung von schräg oben,
- Fig. 2a, b: den Aufbau eines Substrat-Halters in einer Darstellung von schräg oben sowie in seitlicher Schnittdarstellung.
- Fig. 3: ein Flussdiagramm eines Zündfähigkeitstest nach DIN EN ISO 12863:2011-04,
- Fig. 4: ein Flussdiagramm eines erfindungsgemäßen Verfahrens zur Kennzeichnung der rauen Seite eines Filterpapiers (In-Prozess-Verfahren),
- Fig. 5: ein Flussdiagramm eines erfindungsgemäßen Verfahrens zur Kennzeichnung der rauen Seite eines Filterpapiers (Markierung der Rollenware),
- Fig. 6: ein Flussdiagramm eines erfindungsgemäßen Verfahrens zur Kennzeichnung der rauen Seite des Substrates (Markierung der Papierbögen),
- Fig. 7: ein Flussdiagramm eines erfindungsgemäßen Verfahrens zur Kennzeichnung der rauen Seite des Substrates (Markierung der Rundfilter) und
- Fig. 8a-d: unterschiedliche Ausführungsformen erfindungsgemäß markierter Filterpapiere.

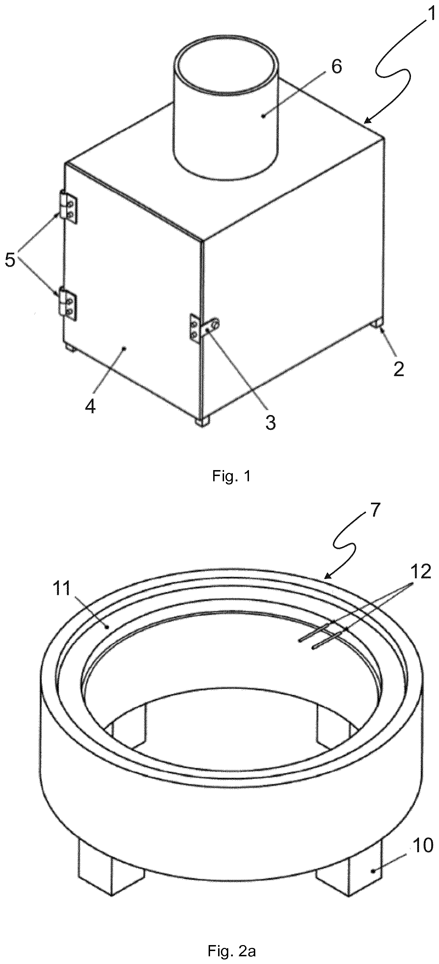

In Fig. 1 ist eine Prüfkammer 1 in Anlehnung an die DIN EN ISO 12863:2011-04 zur Durchführung des erfindungsgemäßen Überprüfungs-Verfahrens in einer Darstellung von schräg oben abgebildet (Quelle der Prüfkammer: DIN EN ISO 12863:2011-04). Die Prüfkammer 1 besitzt eine rechteckige Grundfläche und kann über Füße 2 auf einer Unterlage aufgestellt werden. Die Prüfkammer 1 besteht aus einem starren durchsichtigen Werkstoff und besitzt die Maße 340 ± 25 mm in der Höhe, 292 ± 6 mm in der Breite und 394 ± 6 mm in der Tiefe. Der Innenraum der Prüfkammer 1 ist über eine mit einer Verriegelung 3 verschließbare Frontplatte 4 zugänglich, die an seitlich angebrachten Scharnieren 5 verschwenkbar gehalten ist. Auf der Oberseite der Prüfkammer 1 ist in mittiger Anordnung ein Rauchabzug 6 vorgesehen, der eine Höhe von 165 ± 13 mm und einen Innendurchmesser von 152 ± 6 mm aufweist.

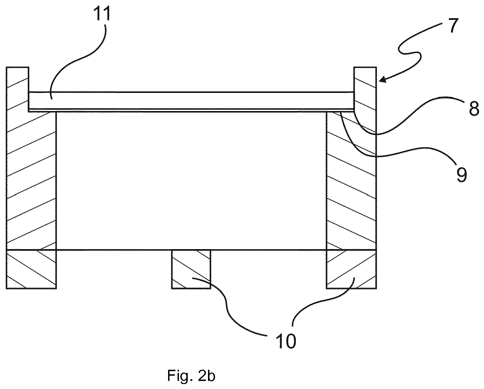

Der Innenraum der Prüfkammer 1 ist zur Aufnahme eines zylindrischen Substrathalters 7 für das Filterpapier und die zu prüfende Zigarette vorgesehen, welcher in Fig. 2a in einer Darstellung von schräg oben und in Fig. 2b in seitlicher Schnittdarstellung abgebildet ist (Quelle des Substrathalters: DIN EN ISO 12863:2011-04). An seiner Oberkante weist der Substrathalter 7 eine zur Innenseite abfallende umlaufende Stufe 8 mit einer Tiefe von 10 ± 2,5 mm, gemessen von der Oberkante des Substrathalters 7, auf. Die Stufe 8 ist konzentrisch angebracht und besitzt einen Durchmesser im Stufenknick von 152 ± 1 mm. Dadurch entsteht ein treppenartiger Absatz, dessen tiefer liegende Fläche dem Filterpapier als umlaufende randseitige Auflage 9 dient. Die umlaufende Auflage 9 besitzt eine Tiefe von etwa 25 mm.

An der Unterseite des Substrathalters 7 sind radial gleichmäßig beabstandet vier Füße 10 mit einer Höhe von jeweils 20 ± 1 mm angebracht. Der Substrathalter 7 besitzt einen Außendurchmesser von 165 ± 1 mm, einen Innendurchmesser von 127 ± 1 mm und eine Wandstärke von etwa 38 mm auf. Die Höhe des Substrathalters ohne die Füße 10 beträgt 50 ± 1 mm.

Um das Filterpapier oder den Stapel von Filterpapieren zu fixieren ist ein Metallkranz 11 vorgesehen, der von oben auf den Rand des in den Substrathalter 7 gelegten Filterpapiers aufgesetzt wird. Der Metallkranz 11 ist als flacher Ring ausgestaltet und besitzt einen Außendurchmesser, der kleiner als der Innendurchmesser der Stufe 8 entspricht, also vorliegend etwa 150 ± 1 mm. Der Metallkranz 11 springt umlaufend von der Innenwand des Substrathalters 7 zurück und weist einen Innendurchmesser von 130 ± 1 mm auf. Die Materialdicke des Metallkranzes 11 liegt bei etwa 6,4 ± 1 mm. Der Metallkranz 11 besteht aus Messing und weist eine Masse des Metallkranzes von etwa 270 g auf.

In den Metallkranz 11 sind parallel zu dessen Flachseiten und etwa mittig in seiner Querschnittsfläche zwei zueinander parallel beabstandete Metallstifte 12 eingelassen, die sich etwa 17 ± 1 mm in das Innere des Metallkranzes 11 erstrecken. Die Metallstifte 12 sind dabei so positioniert, dass eine mittig zwischen und parallel zu den Metallstiften 12 verlaufende gedachte Linie durch den Mittelpunkt des Metallkranzes 11 verläuft. Der Abstand der Metallstifte 12 zueinander ist so gewählt, dass eine Zigarette, die zwischen den Metallstiften 12 eingelegt wird, am seitlichen Wegrollen gehindert, also fixiert ist. Bei einer üblichen Zigarette mit einem Durchmesser von 8 mm beträgt der Abstand der Metallstifte 12 etwa 8,1 ± 1 mm.

In Fig. 3 ist ein Verfahren zur Überprüfung der Zündneigung von Zigarettenpapier gemäß DIN EN ISO 12863:2011-04 vom April 2011 als Ablaufdiagramm dargestellt. Darin muss vom Versuchspersonal zunächst die raue Seite des Filterpapieres bestimmen, was in der Regel händisch geschieht. Bei dem erfindungsgemäßen Verfahren werden hierzu die erfindungsgemäßen markierten Filterpapiere verwendet, die das Auffinden der rauen Oberfläche erheblich erleichtern und ein Fehlbestimmungen nahezu ausschließen.

Anschließend erfolgt die Vorkonditionierung des oder der Filterpapiere. Bei einer 100-Blatt-Schachtel wird diese bei offenem Deckel für mindestens sieben Tage (7 * 24 h) vor der Prüfung in einer Umgebung mit einer relativen Luftfeuchtigkeit von 55 ± 5 % und einer Temperatur von 23 ± 3 °C aufbewahrt. Bei der Verwendung von Filterpapierstapeln werden dementsprechende Sets, also Stapel von 3, 10 oder 15 markierten Filterpapieren für mindestens 8 h vor der Prüfung in einer Umgebung mit einer relativen Luftfeuchtigkeit von 55 ± 5 % und einer Temperatur von 23 ± 3 °C aufbewahrt. Die Standardabweichung von 5 Sätzen mit je 15 Blättern des konditionierten Filterpapiers muss 26,1 ± 0,5 g betragen, wobei die Standardabweichung der fünf Wägungen nicht mehr als 0,3 g beträgt.

Das eigentliche Verfahren zur Überprüfung der Zündneigung von Zigarettenpapier verläuft dann in der Weise, dass das vorkonditionierte Filterpapier auf den Substrathalter 7 aufgelegt wird, wobei die raue Oberfläche des Filterpapieres nach oben zeigt. Anschließend wird der Metallkranz 11 auf das Filterpapier gelegt, wobei das Filterpapier durch das Gewicht des Metallkranzes 11 randseitig fixiert wird. Bei der Verwendung von Filterpapierstapeln werde diese durch den Metallkranz 11 zusammengedrückt.

Die dem Test zugeführte Zigarette wird vor dem Anzünden vorkonditioniert, indem diese mindestens 24 h vor der Prüfung in einer Umgebung mit einer relativen Luftfeuchtigkeit von 55 ± 5 % und einer Temperatur von 23 ± 3 °C aufbewahrt wird. Hierdurch können Schwankungen im Abbrennverhalten kompensiert werden, die aus einer möglichen unterschiedlichen Vorlagerung herrühren könnten. Die zu untersuchende Zigarette wird vor Anzünden mit einem Bleistift bei 5 ± 1 mm und 15 ± 1 mm vom anzuzündenden Zigarettenende ausgehend markiert. Anschließend wird die Zigarette entzündet und erst dann mit der Naht des Zigarettenpapiers nach oben auf die raue Oberfläche des eingespannten Filterpapiers beziehungsweise den Filterpapierstapel gelegt, wenn die Glut die 15 ± 1 mm Markierung ausgehend vom angezündeten Zigarettenende erreicht. Die markierte Zigarette wird dabei zwischen die Metallstifte 12 des Metallkranzes 11 gelegt und auf diese Weise fixiert, also insbesondere am Wegrollen gehindert.

Das Abbrennverhalten der Zigarette wird nach folgenden Kriterien ausgewertet:
A) vollständiges Abbrennen der Zigarette;
B) unvollständiges Abbrennen der Zigarette;
C) Selbstlöschung, wenn die Zigarette nach dem Anzünden erlischt, bevor die 15 ± 1 mm vom anzuzündenden Zigarettenende ausgehende Markierung der Zigarette erreicht ist, wird als A) gewertet mit dem Vermerk, dass dies vor dem Auflegen auf die raue Oberfläche des Zellulosefilterpapiers geschehen ist;
D) Wenn der Rauch der abbrennenden Zigarette auch nach Überprüfung der Kammer und des Abluftsystems nicht laminar ist, wird dieses Ergebnis festgehalten.

Der Test wird mit wenigstens 40 Zigaretten gleichen Typs wiederholt, um eine ausreichende Basis für eine statistische Auswertung des Abbrennverhaltens zu erlangen.

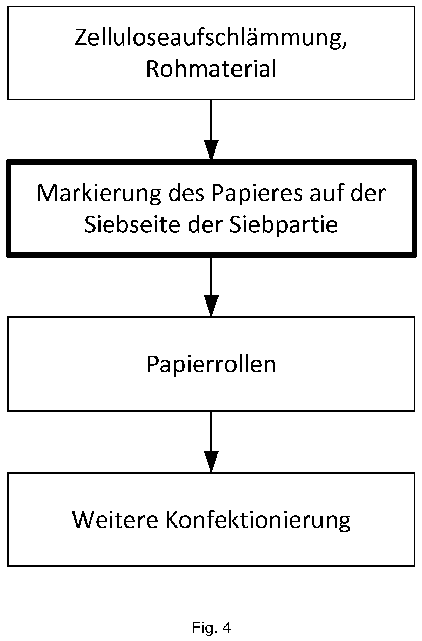

In den Fign. 4 bis 7 sind vier verschiedene Ausführungsformen des erfindungsgemäßen Verfahrens zur Herstellung eines erfindungsgemäßen markierten Zellulose-Filterpapiers zur Überprüfung der Zündneigung von Zigarettenpapier in Form von Ablaufdiagrammen dargestellt.

Bei dem in Fig. 4 dargestellten Ablaufdiagramm wird das Filterpapier noch während der Herstellung auf der Siebseite der Siebpartie markiert, beispielsweise mittels eines echten Wasserzeichens. Die Wasserzeichen sind dabei in der Weise angeordnet, dass sie nach dem Durchlaufen des gesamten Produktionsprozesses bis hin zum fertigen Rundfilter-Papier an der gewünschten Stelle jedes einzelnen Filterpapieres angeordnet sind.

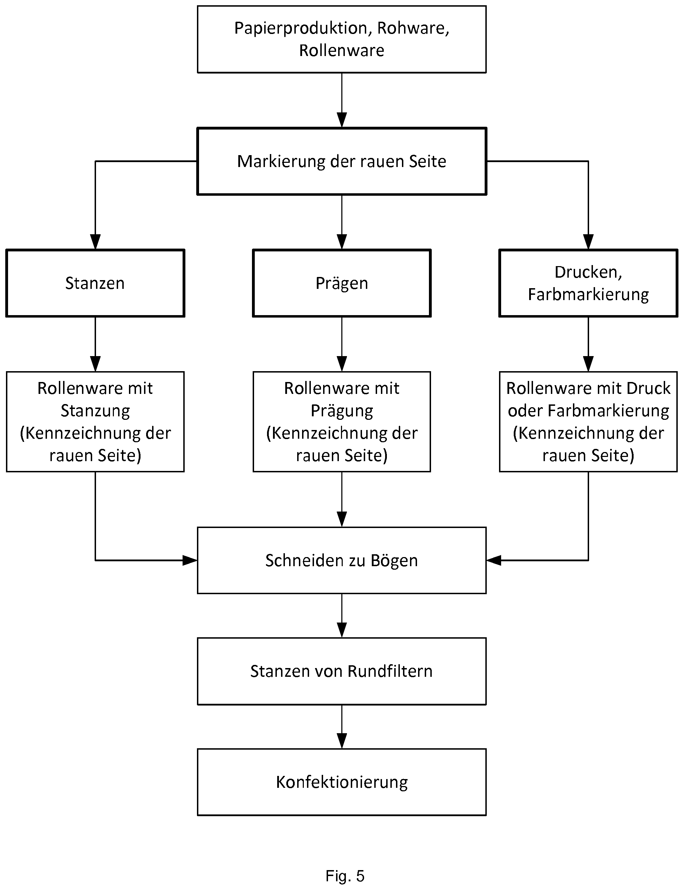

In Fig. 5 wird eine weitere Möglichkeit der Herstellung von erfindungsgemäßen markiertem Filterpapier dargestellt. Bei diesem Verfahren wird das Filterpapier dem Markierungsprozess als Rollenware zugeführt. Bei der Rollenware ist die Lage der rauen Oberfläche entweder vom Produktionsprozess her schon bekannt oder kann mithilfe eines Messverfahrens einmalig für die gesamte Rolle bestimmt werden, sodass die Orientierung für den weiteren Produktionsprozess bekannt ist. Die Markierung erfolgt dann an der Rollenware durch Stanzen, Prägen oder Drucken an denjenigen Positionen, an denen später bei dem fertigen Rundfilter die Markierung gewünscht ist. Anschließend wird die markierte Rollenware zu Bögen geschnitten, von denen dann die Rundfilter ausgestanzt werden.

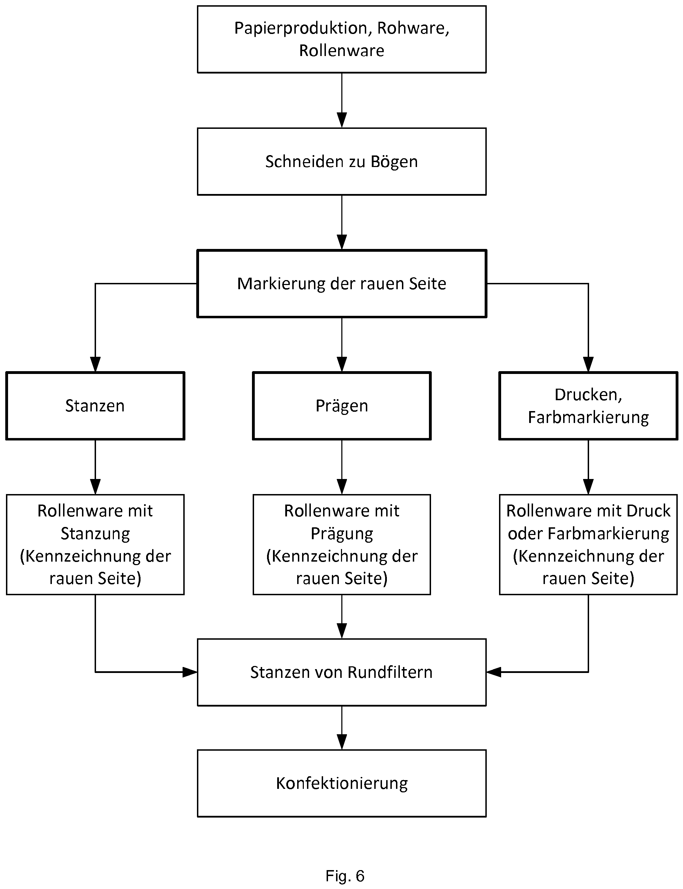

In Fig. 6 ist ein Ablaufdiagramm einer dritten Ausgestaltung eines erfindungsgemäßen Herstellungsverfahrens für markierte Filterpapiere abgebildet. Bei dieser Verfahrensvariante erfolgt die Markierung nach dem Zuschneiden der Rollenware zu Bögen. Auch in diesem Fall ist die Lage der rauen Oberfläche entweder vom Produktionsprozess her bekannt oder kann für die gesamte Rolle vor dem Zuschnitt zu Bögen bestimmt werden. Wenn Bögen angeliefert werden, kann die raue Oberfläche auch für jeden Bogen einzeln bestimmt werden. Auf den Bögen werden dann die jeweiligen Markierungen durch Stanzen, Prägen oder Drucken an entsprechend gewünschter Position der später erzeugten Rundfilter angebracht. Die Rundfilter werden dann aus den Bögen ausgestanzt und verpackt.

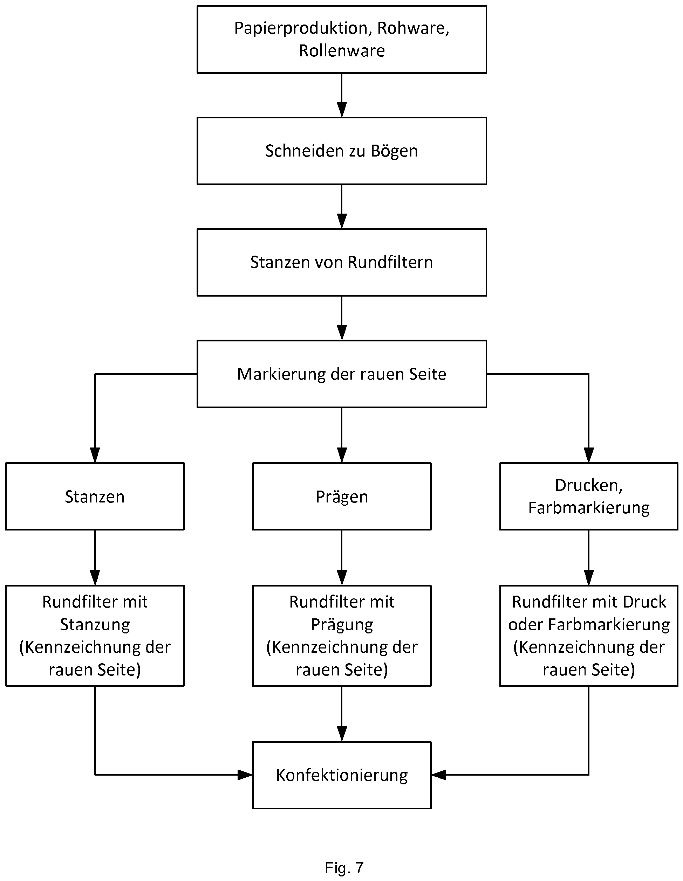

In Fig. 7 ist eine vierte Ausführungsform eines erfindungsgemäßen Herstellungsverfahrens zur Erzeugung markierter Papierfilter dargestellt. Bei dem hier gezeigten Ablaufdiagramm erfolgt nunmehr die Markierung der Filterpapiere, nachdem diese aus den Bögen ausgestanzt sind. Auch in diesem Falle ist die Lage der rauen Seite der in den Produktionsprozess eingeschleusten Rollenware entweder von der Herstellung bekannt oder kann zuvor anhand eines einzigen Versuchs für die gesamte Papierrolle bestimmt werden. Die Markierung der gestanzten Rundfilter erfolgt dann über Stanzen, Prägen oder Drucken. Da die Lage der rauen Oberfläche über den gesamten Produktionsprozess bekannt ist, ist es hierbei nicht erforderlich, vor dem Anbringen der Markierung die raue Oberfläche eines jeden Rundfilters zu bestimmen. Das Anbringen der Markierung auf gestanzten Rundfiltern hat den Vorteil, dass die Lager der Markierung(en) mit einer höheren Präzision definiert werden kann als bei den anderen vorgenannten Verfahren.

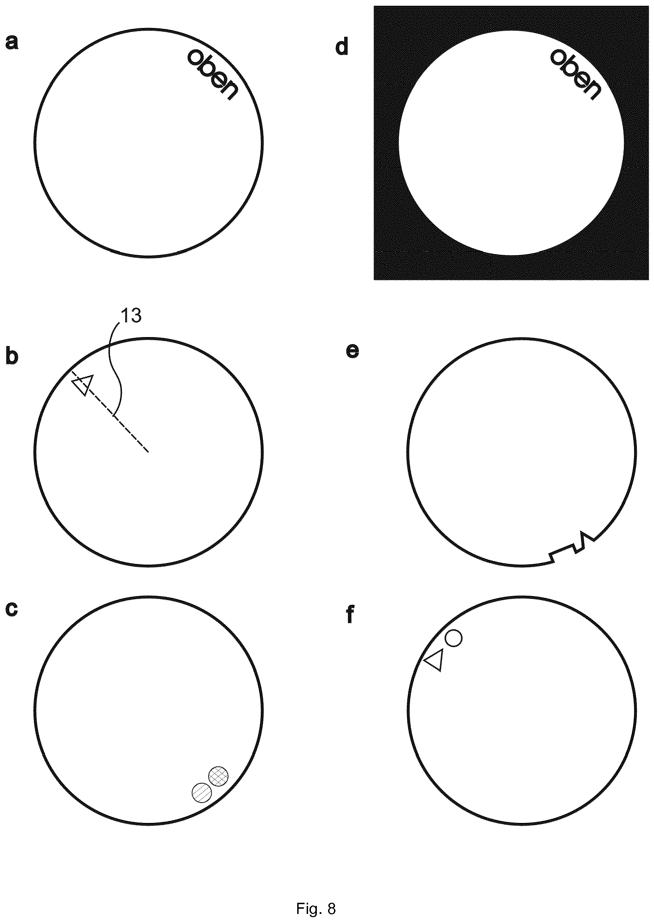

In den Fign. 8a bis f sind verschiedene Ausführungsformen erfindungsgemäß markierter Rundfilter abgebildet. Bei der in Fig. 8a dargestellten Ausführungsform ist auf dem Zellulose-Rundfilter auf der rauen Oberfläche eine Markierung "oben" angebracht. Diese kann nur von der richtigen Seite gelesen werden. Wenn die Markierung von der Rückseite ebenfalls grundsätzlich sichtbar ist, wie beispielsweise bei einem Wasserzeichen oder einer Prägung, kann das Wort "oben" von der glatten Seite jedoch nur spiegelverkehrt gelesen werden, so dass die Identifizierung der "richtigen" Seite, nämlich der rauen Oberfläche, für ein Versuchspersonal leicht zu bewerkstelligen ist.

In Fig. 8b ist eine Ausführungsform eines markierten Filterpapiers dargestellt, bei dem ein unsymmetrisches Symbol in Form eines Dreiecks aufgebracht wird. Die Unsymmetrie ergibt sich hierbei daraus, dass das Zeichen keine Spiegelsymmetrie im Hinblick auf eine Radiuslinie 13 besitzt, die sich vom Mittelpunkt des Rundfilters mittig durch die Markierung, also das Dreieck, hindurchzieht. Das Dreieck zeigt bei der in Fig. 8b dargestellten Ausführungsform in Richtung des Uhrzeigersinns. Wenn diese Markierung auch von der Rückseite des Filterpapiers sichtbar ist, würde sie in diesem Fall gegen Uhrzeigersinn weisen und damit auch bei einer beidseitig sichtbaren Markierung dem Versuchspersonal die raue Filterpapier-Oberfläche eindeutig anzeigen.

In Fig. 8c ist eine weitere Ausgestaltung eines erfindungsgemäß markierten Filterpapiers dargestellt, bei dem gefärbte Zeichen aufgedruckt sind (vorliegend als unterschiedliche Schraffur dargestellt), die die raue Oberfläche des Filterpapiers kennzeichnen.

In Fig. 8d ist ein analog zu Fig. 8a markiertes Filterpapier abgebildet, wobei der Schriftzug "oben" in diesem Fall aus dem Papier ausgestanzt wurde. Beim Betrachten von der rauen Seite erscheint der ausgestanzte Schriftzug in Klartext (der Hintergrund ist durch die Stanzung hindurch zu sehen).

In Fig. 8e ist eine weitere Ausführungsform eines erfindungsgemäß markierten Filterpapiers dargestellt. Darin sind geometrische Figuren aus dem Rand des Filterpapiers ausgestanzt, nämlich vorliegend ein Rechteck und ein Keil. Die raue Oberfläche des Filterpapiers wird bei der hier dargestellten Ausführungsform dadurch gekennzeichnet, dass die rechteckige Stanzung im Uhrzeigersinn vor der Keil-förmigen Stanzung liegt. Diese Ausgestaltung hat im Übrigen den weiteren Vorteil, dass bei der Verwendung von Filterpapier-Stapeln die korrekte Zusammenstellung eines jeden Stapels anhand der Stanzungen direkt überprüft werden kann, wenn die Filterpapiere in der Weise aufeinandergelegt werden, dass die Stanzungen jeweils übereinander zu liegen kommen. Würde ein Filterpapier falsch herum liegen, würde dies sofort auffallen, da dann nicht alle Stanzungen durchgängig zu sehen wären.

In Fig. 8f ist eine sechste Ausführungsform eines erfindungsgemäßen markierten Zellulose-Rundfilter-Papiers dargestellt. Darin sind auf dem Rundfilter im Randbereich zwei geometrische Figuren aufgebracht, nämlich ein Dreieck, dessen eine Spitze auf einen ebenfalls aufgebrachten Kreis zeigt. Die raue Oberfläche ist bei Ausführungsformen, bei denen diese beiden Symbole auf beiden Seiten des Filterpapieres sichtbar sind, dadurch erkennbar, dass auf der rauen Seite der Kreis im Uhrzeigersinn vor dem Dreieck liegt und das Dreieck in Richtung des Uhrzeigersinns weist.

### Bezugszeichenliste:

- 1: Prüfkammer
- 2: Fuß
- 3: Verriegelung
- 4: Frontplatte
- 5: Scharnier
- 6: Rauchabzug
- 7: Substrathalter
- 8: Stufe
- 9: Auflage
- 10: Fuß
- 11: Metallkranz
- 12: Stift
- 13: Radiuslinie

## Patentansprüche

1. Zellulose-Filterpapier zur Überprüfung der Zündneigung von Zigarettenpapier, **dadurch gekennzeichnet, dass** das Filterpapier mit einer Markierung versehen ist, mit der die raue von der glatten Oberfläche des Filterpapiers unterschieden werden kann, wobei die Rauigkeit gemäß DIN 53108:1995-01 definiert ist.

2. Filterpapier nach Anspruch 1, **dadurch gekennzeichnet, dass** das Filterpapier ein einlagiger Rundfilter mit einem Durchmesser im Bereich von 130 mm bis 170 mm ist, insbesondere mit einem Durchmesser von 150 ± 2 mm.

3. Filterpapier nach Anspruch 1 oder 2, **dadurch gekennzeichnet, dass** die Markierung eine Unterscheidung der rauen von der glatten Oberfläche des Filterpapieres auf haptischem Wege und/oder mit dem normalsichtigen menschlichen Auge erlaubt.

4. Filterpapier gemäß einem der vorstehenden Ansprüche, **dadurch gekennzeichnet, dass** die Markierung des Filterpapiers die raue Oberfläche des Filterpapiers kennzeichnet.

5. Filterpapier gemäß einem der vorstehenden Ansprüche, **dadurch gekennzeichnet, dass** die Markierung des Filterpapiers ein Wasserzeichen, ein halbechtes Wasserzeichen, eine Prägung, ein Druck und/ oder eine Stanzung umfasst, wobei die Markierung insbesondere außerhalb des Mittelpunkt des Filterpapiers positioniert ist, vorzugsweise in einem Randbereich des Filterpapiers.

6. Filterpapier gemäß einem der vorstehenden Ansprüche, **dadurch gekennzeichnet, dass** die Markierung ein Wort oder eine Wortfolge irgendeiner Sprache umfasst oder daraus besteht, das/die auf der rauen Oberfläche des Filterpapiers lesbar ist.

7. Filterpapier gemäß einem der vorstehenden Ansprüche, **dadurch gekennzeichnet, dass** die Markierung ein Symbol umfasst oder daraus besteht, wobei die Markierung insbesondere mindestens zwei geometrische Symbole umfasst oder daraus besteht, die sich vorzugsweise in ihrer Form, Farbe und/ oder Größe voneinander unterscheiden.

8. Verfahren zur Herstellung eines Zellulose-Filterpapiers nach einem der Ansprüche 1 bis 7, **dadurch gekennzeichnet, dass** das Filterpapier mit einer Markierung versehen wird, mit der die raue von der glatten Oberfläche des Filterpapiers unterschieden werden kann.

9. Verfahren zur Überprüfung der Zündneigung von Zigarettenpapier umfassend die Schritte:
a) Vorkonditionierung eines runden Zellulose-Filterpapiers, das eine raue und eine glatte Oberfläche aufweist;
b) horizontales Auflegen des vorkonditionierten Filterpapieres auf einen Substrathalter (7), wobei die raue Oberfläche des Filterpapieres nach oben zeigt,
c) danach Auflegen eines Metallkranzes (11) auf das Filterpapier, wobei der Metallkranz (11) zwei parallel zueinander beabstandete Metallstifte (12) zur Fixierung einer entzündeten Zigarette aufweist, die in das Kranzinnere hineinragen und zueinander einen Abstand und eine Länge aufweisen, um das nicht entzündete Ende einer Zigarette zu fixieren;
d) anschließend Auflegen einer an einem Ende entzündeten Zigarette mit der Naht des Zigarettenpapiers nach oben auf die raue Oberfläche des Filterpapieres, wobei das nicht entzündete Ende der Zigarette zwischen den Metallstiften (12) fixiert wird und
e) Ermitteln des Abbrennverhaltens der Zigarette,
**dadurch gekennzeichnet, dass** das Filterpapier ein Zellulose-Filterpapier nach einem der Ansprüche 1 bis 7 ist, wobei der Schritt e) nach folgenden Kriterien ausgewertet wird:
A) vollständiges Abbrennen der Zigarette;
B) unvollständiges Abbrennen der Zigarette,
wobei A) bedeutet, dass eine Zigarette weiterbrennt, bis die Vorderkante des Mundstückpapiers bei Filterzigaretten oder bei Zigaretten ohne Filter die Metallstifte (12) erreicht ist und wobei B) bedeutet, dass die Zigarette erlischt, bevor die Vorderkante des Mundstückpapiers bei Filterzigaretten oder bei Zigaretten ohne Filter die Metallstifte (12) erreicht sind.

10. Verfahren nach Anspruch 9, **dadurch gekennzeichnet, dass** das Verfahren die weiteren Schritte umfasst:
f) wenigstens 39 maliges Wiederholen der Schritte a) bis e) und
g) Ermitteln des Anteils der wenigstens 40 Bestimmungen, bei denen das Ergebnis A) lautet.

11. Verfahren nach einem der Ansprüche 9 oder 10, **dadurch gekennzeichnet, dass** im Schritt a) eine Vielzahl von Filterpapieren vorkonditioniert wird und im Schritt b) ein Stapel der vorkonditionierten Filterpapiere auf den Substrathalter (7) aufgelegt wird, wobei die raue Oberfläche der Filterpapiere des Stapels nach oben zeigen und der Stapel wenigstens 3, insbesondere 10 oder 15 Filterpapiere umfasst.

12. Verfahren nach einem der Ansprüche 9 bis 11, **dadurch gekennzeichnet, dass** die Vorkonditionierung des Filterpapiers im Schritt a) in der Weise durchgeführt wird, dass eine 100-Blatt-Schachtel mit markierten Filterpapier bei offenem Deckel für mindestens sieben ganze Tage (7 * 24 h) in einer Umgebung mit einer relativen Luftfeuchtigkeit von 55 ± 5 % und einer Temperatur von 23 ± 3 °C aufbewahrt wird oder Sets von 3, 10 oder 15 Filterpapieren für mindestens 8 h vor der Prüfung in einer Umgebung mit einer relativen Luftfeuchtigkeit von 55 ± 5 % und einer Temperatur von 23 ± 3 °C aufbewahrt werden.

13. Verfahren nach einem der Ansprüche 9 bis 12, **dadurch gekennzeichnet, dass** das Verfahren gemäß DIN EN ISO 12863 aus 2010 oder DIN EN ISO 12863:2011-04 durchgeführt wird.

14. Kit umfassend eine Vielzahl von Zellulose-Filterpapieren nach einem der Ansprüche 1 bis 7 oder wenigstens einen Stapel von 3, 10 oder 15 markierten Zellulose-Filterpapieren nach einem der Ansprüche 1 bis 7, optional eine Information, wie die raue Oberfläche des Zellulose-Filterpapieres anhand der Markierung zu ermitteln ist und optional eine Versuchsdurchführung der DIN EN ISO 12863 und/ oder DIN EN ISO 12863:2011-04.

15. Kit gemäß Anspruch 14, **dadurch gekennzeichnet, dass** die Ausrichtung aller rauen Oberflächen der Zellulose-Filterpapiere eines Stapels identisch ist.

16. Kit gemäß Anspruch 14 oder 15, **dadurch gekennzeichnet, dass** darin wenigstens 5 oder wenigstens 10 Stapel enthalten sind.

## Claims

1. Cellulose filter paper to check the ignition propensity of cigarette paper, **wherein** the filter paper is provided with a marking with which the smooth surface of the filter paper can be distinguished from the rough surface, wherein the roughness is defined according to DIN 53108:1995-01.

2. Filter paper according to claim 1, **wherein** the filter paper is a single-layer round filter having a diameter in the range of from 130 mm to 170 mm, in particular having a diameter of 150 ± 2 mm.

3. Filter paper according to claim 1 or 2, **wherein** the marking allows to discriminate between the rough and the smooth surface of the filter paper by means of haptic sensation and/or with the normal-sighted human eye.

4. Filter paper according to one of the proceeding claims, **wherein** the marking of the filter paper characterizes the rough surface of the filter paper.

5. Filter paper according to one of the proceeding claims, **wherein** the marking of the filter paper comprises a watermark, a semi-fragile watermark, an embossing, a print and/or a punch, whereby the marking is positioned in particular outside of the center point of the filter paper, preferably in an edge region of the filter paper.

6. Filter paper according to one of the proceeding claims, **wherein** the marking comprises or consists of a word or phrase in any language that is legible on the rough surface of the filter paper.

7. Filter paper according to one of the proceeding claims, **wherein** the marking comprises or consists of a symbol, whereby the marking comprises in particular at least two geometrical symbols that differ from each other with respect to their shape, color and/or size.

8. Method for producing a cellulose filter paper according to one of the claims 1 to 7, **wherein** the filter paper is provided with a marking with which the smooth surface of the filter paper can be distinguished from the rough surface.

9. Method for checking the ignition propensity of cigarette paper comprising the steps:
a) Preconditioning a round cellulose filter paper having a rough and a smooth surface;
b) Horizontally placing the pre-conditioned filter paper onto a substrate holder (7), whereby the rough surface of the filter paper faces upward,
c) Subsequently placing a metal ring (11) onto the filter paper, whereby the metal ring (11) has two metal pins (12) spaced in parallel to each other for fixing an ignited cigarette that protrude into the interior of the ring and have a spacing to each other and a length that allows the unlit end of a cigarette to be fixed;
d) Subsequently placing a cigarette ignited at one end with the seam of the cigarette paper facing upward onto the rough surface of the filter paper, whereby the non-lit end of the cigarette is fixed between the metal pins (12) and
e) Determining the burning behavior of the cigarette,
**wherein** the filter paper is the cellulose filter paper according to one of claims 1 to 7, whereby step e) is analyzed according to the following criteria:
A) complete burning of the cigarette;
B) incomplete burning of the cigarette,
whereby A) means that when filter cigarettes are used a cigarette continues to burn until the front edge of the tipping paper is reached, or when cigarettes without filter are used until the metal pins (12) are reached, and B) means that when filter cigarettes are used the cigarette extinguishes before the front edge of the tipping paper is reached or when cigarettes without filter are used before the metal pins (12) are reached.

10. Method according to claim9, **wherein** the method comprises the additional steps;
f) At least 39 repetitions of steps a) to e) and
g) Determining the proportion of the at least 40 tests in which the result was A).

11. Method according to one of claims 9 or 10, **wherein** a plurality of filter papers are preconditioned in step a) and a stack of the preconditioned filter papers are placed on the substrate holder (7) in step b), whereby the rough surfaces of the filter papers of the stack face upward and the stack comprises at least 3, in particular 10 or 15 filter papers.

12. Method according to one of the claims 9 to 11, **wherein** the preconditioning of the filter paper in step a) is performed in such a manner that a 100-sheet box containing marked filter paper is stored with an open lid for at least seven days (7*24 h) in an environment with a relative humidity of 55 ± 5% and a temperature of 23 ± 3 °C., or sets of 3, 10 or 15 filter papers are stored for at least 8 h before the test in an environment with a relative humidity of 55 ± 5% and a temperature of 23 ± 3 °C.

13. Method according to one of the claims 9 to 12, **wherein** the method is performed according to DIN EN ISO 12863 of 2010 or DIN EN ISO 12863:2011-04.

14. Kit comprising a plurality of cellulose filter papers according to one of claims 1 to 7 or at least a stack of 3, 10 or 15 marked cellulose filter papers according to one of the claims 1 to 7, optionally information on how the rough surface of the cellulose filter paper can be determined by means of the marking, and optionally an experimental procedure according to DIN EN ISO 12863 and/or DIN EN ISO 12863:2011-04.

15. Kit according to claim 14, **wherein** the orientation of all rough surfaces of the cellulose filter papers of a stack are identical.

16. Kit according to claim 14 or 15, **wherein** at least 5 stacks are contained therein.

## Revendications

1. Papier-filtre en cellulose pour contrôler la tendance à l'inflammation du papier à cigarette, **caractérisé en ce que** le papier-filtre est muni d'un marquage permettant de distinguer la surface rugueuse de la surface lisse du papier-filtre.

2. Papier-filtre selon la revendication 1, **caractérisé en ce que** le papier filtre est un filtre rond monocouche ayant un diamètre compris entre 130 mm et 170 mm, en particulier un diamètre de 150 ± 2 mm.

3. Papier-filtre selon la revendication 1 ou 2, **caractérisé en ce que** le marquage permet de distinguer le rugueux de la surface lisse du papier filtre par des moyens haptiques et/ou à l'oeil humain.

4. Papier-filtre selon l'une des revendications précédentes, **caractérisé en ce que** le marquage du papier-filtre marque la surface rugueuse du papier filtre.

5. Papier-filtre selon l'une des revendications précédentes, **caractérisé en ce que** le marquage du papier-filtre comprend un filigrane, un filigrane demi-authentique, un gaufrage, une impression et/ou un poinçonnage, le marquage en particulier étant placé en dehors du centre du papier-filtre, de préférence dans une zone périphérique du papier-filtre.

6. Papier-filtre selon l'une des revendications précédentes, **caractérisé en ce que** le marquage comprend ou consiste en un mot ou une expression de toute langue lisible sur la surface rugueuse du papier-filtre.

7. Papier-filtre selon l'une des revendications précédentes, **caractérisé en ce que** le marquage comprend ou consiste en un symbole, le marquage comprenant ou consistant notamment en au moins deux symboles géométriques qui diffèrent de préférence l'un de l'autre en forme, couleur et/ou taille.

8. Procédé de fabrication d'un papier-filtre en cellulose selon l'une quelconque des revendications 1 à 7, **caractérisé en ce que** le papier-filtre est muni d'un marquage qui distingue la surface rugueuse de la surface lisse du papier-filtre.

9. Procédé pour vérifier la tendance à l'allumage du papier à cigarette, comprenant les étapes suivantes:
a) le préconditionnement d'un papier-filtre rond en cellulose ayant une surface rugueuse et lisse;
b) placer horizontalement le papier-filtre préconditionné sur un support de substrat (7) avec la surface rugueuse du papier-filtre tournée vers le haut,
c) placer ensuite un anneau métallique (11) sur le papier-filtre, l'anneau métallique (11) ayant deux broches métalliques (12) espacées parallèlement l'une de l'autre pour fixer une cigarette allumé, qui font saillie dans l'intérieur de l'anneau et sont écartées et ont une longueur pour fixer la fin non allumée d'une cigarette;
d) placer ensuite une cigarette qui est allumée à une extrémité avec le joint du papier à cigarette tourné vers le haut sur la surface rugueuse du papier filtre, l'extrémité non allumée de la cigarette étant fixée entre les broches métalliques (12), et
e) déterminer le comportement de brûlure de la cigarette,
**caractérisé en ce que** le papier-filtre est un papier-filtre en cellulose selon l'une des revendications 1 à 7, l'étape e) étant évaluée selon les critères suivants:
(A) la brûlure complète de la cigarette;
(B) brûlure incomplète de la cigarette,
A) signifiant qu'une cigarette continue à brûler jusqu'à ce que le bord d'attaque du papier de l'embout buccal dans le cas de cigarettes à filtre est atteint ou les broches métalliques (12) dans le cas de cigarettes sans filtre sont atteintes, et B) signifiant que la cigarette s'éteint avant que le bord d'attaque du papier de l'embout buccal dans le cas de cigarettes à filtre ou les broches métalliques dans le cas de cigarettes sans filtre sont atteintes.

10. Procédé selon la revendication 9, **caractérisé en ce que** le procédé comprend les étapes supplémentaires consistant à : f) répéter les étapes a) à e) au moins 39 fois, et g) déterminer la proportion des au moins 40 déterminations pour lesquelles le résultat est A).

11. Procédé selon l'une des revendications 9 ou 10, **caractérisé en ce que** dans l'étape a), une pluralité de papiers-filtres est préconditionné et dans l'étape b) une pile des papiers-filtres préconditionnés est placée sur le support de substrat (7), la surface rugueuse des papiers filtres de la pile étant tournée vers le haut et la pile comprenant au moins 3, en particulier 10 ou 15 papiers filtres.

12. Procédé selon l'une quelconque des revendications 9 à 11, **caractérisé en ce que** le préconditionnement du papier-filtre dans l'étape a) est effectué en stockant une boîte de 100 feuilles de papier-filtre marqué avec le couvercle ouvert pendant au moins sept jours entiers (7 * 24 h) dans un environnement ayant une humidité relative de 55 ± 5% et une température de 23 ± 3°C ou par lots de 3, 10 ou 15 papiers filtres pendant au moins 8 heures avant l'essai dans un environnement ayant une humidité relative de 55 ± 5 % et une température de 23 ± 3 °C.

13. Procédé selon l'une des revendications 9 à 12, **caractérisé en ce que** le procédé est exécuté selon DIN EN ISO 12863 à partir de 2010 ou selon DIN EN ISO 12863:201 1 -04.

14. Kit comprenant une pluralité de papiers-filtres en cellulose selon l'une des revendications 1 à 7 ou au moins une pile de 3, 10 ou 15 papiers-filtres cellulose marqués selon l'une des revendications 1 à 7, éventuellement des informations sur la manière dont la surface rugueuse du papier-filtre cellulose doit être déterminée en fonction de la marquage et éventuellement une performance expérimentale selon DIN EN ISO 12863 et/ou DIN EN ISO 12863:201 1 -04.

15. Kit selon la revendication 14, **caractérisé en ce que** l'alignement de toutes les surfaces rugueuses des papiers-filtres en cellulose d'une pile est identique.

16. Kit selon la revendication 14 ou 15, **caractérisé en ce qu'**il contient au moins 5 ou au moins 10 piles.
